Europäisches Patentamt

European Patent Office

Office européen des brevets

(19)

(11) Publication number: **0 465 132 A2**

(12) **EUROPEAN PATENT APPLICATION**

(21) Application number: **91305835.0**

(22) Date of filing: **27.06.91**

(51) Int. Cl.$^5$: **C12N 15/54,** C12N 9/12, C12N 15/75, C12P 19/38, C12N 1/20, // (C12N1/20, C12R1:125)

(30) Priority: **03.07.90 JP 176632/90**

(43) Date of publication of application: **08.01.92 Bulletin 92/02**

(84) Designated Contracting States: **AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku Osaka 541 (JP)**

(72) Inventor: **Miyagawa, Kenichiro**
**6-11, Higashitokiwadai 6-chome, Toyono-cho Toyono-gun, Osaka 663-01 (JP)**
Inventor: **Kanzaki, Naoyuki**
**17-201, 8 Kuragauchi 2-chome Ibaraki, Osaka 567 (JP)**

(74) Representative: **Horton, Sophie Emma et al Elkington and Fife, Prospect House, 8 Pembroke Road Sevenoaks, Kent TN13 1XR (GB)**

(54) **DNA and its use.**

(57) There is disclosed a recombinant DNA comprising DNA containing a phosphoribosyl pyrophosphate synthetase structural gene of a microorganism of the genus Bacillus, DNA containing a ribosome binding site and DNA containing a promoter sequence being capable of expression in a microorganism of the genus Bacillus, these DNA sequences being modified so as to enhance the expression of the phosphoribosyl pyrophosphate synthetase gene. The DNA containing the promoter sequence is located closely to and upstream from the DNA containing the ribosome binding site. The vector containing the recombinant DNA, a novel microorganism of the genus Bacillus capable of producing nucleic acid related substances which are transformed with the recombinant DNA or vector, and a process for producing nucleic acid related substances using the microorganism of the genus Bacillus are also disclosed.

EP 0 465 132 A2

Fig.3.

## FIELD OF THE INVENTION

The present invention relates to a process for producing nucleic acid related substances such as inosine and/or guanosine by fermentation, a novel microorganism used for the production of the substances and a novel recombinant DNA to be used for the derivation of the novel microorganism.

## BACKGROUND OF THE INVENTION

For the production of nucleic acid related substances, the fermentative production using microorganisms is considered to be advantageous, and various processes have been proposed and carried out.

As a result of various studies on nucleic acid fermentations until now, it has been known that nucleic acids or related substances thereof are primary metabolites which are essential to the living cell and generally biosynthesized by microorganisms but, at the same time, the amounts thereof produced are skillfully regulated to prevent producing excessively. Based on such knowledge, for producing nucleic acids or related substances thereof excessively, it is considered to be advantageous to use a mutant wherein regulation of the biosynthetic pathway by end products is released.

For example, it has been known that the production of inosine and/or guanosine,which are purine nucleotide related substances,undergoes negative feedback regulation by end products of the purine nucleotide biosynthesis pathway such as adenine related substances (e.g. adenosine or its phosphorylated compound such as 5′-adenylic acid), or guanine related substances (e.g. guanosine or its phosphorylated compound such as 5′-guanylic acid).

Then, usually, in the fermentative production of a purine nucleotide related substance, the amount of adenine or its related substances in a culture medium is limited to a low concentration to avoid the negative regulation of the biosynthesis of the purine nucleotide related substance by adenine or its related substances and, further, an adenine-requiring mutant which lacks adenylosuccinate synthetase is used (Y. Nakao, Microbial Technology, Vol. 1, p 311-354, edited by H. J. Pepper and D. Perlman, Academic Press, New York). Further, it has been known to employ processes using bacteria belonging to the genera Bacillus (U.S. Patent 3,960,661, Japanese Patent Publication No. 41915/1982, U.S. Patent No. 4,701,413, U.S. Patent No. 3,912,587 and U.S. Patent No. 4,283,346) and Brevibacterium (U.S. Patent No. 3,736,228 and Japanese Patent Publication No. 17592/1983) provided with resistance to various drugs such as analogues of nucleic acid and the like in addition to adenine-requiring for increased production of inosine and/or guanosine.

In addition, a process for producing nucleic acid related substances which comprises inserting a cloned gene of an enzyme concerning biosynthesis of a nucleic acid to a vector and introducing it into a host to excessively produce nucleic acid related substance by a so-called gene amplification effect has also been proposed (Japanese Patent Laid Open Publication Nos. 28470/1984 and 156388/1985).

However, many mutants involved in purine nucleotide biosynthesis and its degradation have been obtained by conventional methods, but they are unsatisfactory and a more improved mutant is desired because nucleic acid related substances are important as raw materials for food and pharmaceutical industries and the like. Therefore, a more advantageous production method is desired.

## OBJECTS OF THE INVENTION

Under these circumstances, the present inventors have intensively investigated factors which are concerned in the improved production of nucleic acid related substances. As a result, it has been found that an increase in the activity of an enzyme, phosphoribosyl pyrophosphate synthetase (PRPP synthetase) which is concerned in the formation of phosphoribosyl pyrophosphate (PRPP) which is an intermediate of the biosynthesis of nucleic acid related substances is of importance. Namely, the present inventors have succeeded in the construction of a novel recombinant DNA having a structure in which the prs gene coding for PRPP synthetase is located downstream of a promoter which is expected to be that for high expression of the synthetase. Further, by using the recombinant DNA, the present inventors have succeeded in the derivation of a novel microorganism, wherein the PRPP synthetase gene is highly and stably expressed, by transforming a recipient suitably selected from microorganisms of the genus Bacillus.

Then, it has been found that the novel microorganism derived from a recipient, a microorganism selected from microorganisms of the genus Bacillus, by using this technique can constantly accumulate a large amount of nucleic acid related substances.

The present invention has been accomplished based on the above finding.

Namely, one object of the present invention is to provide a novel recombinant DNA which is useful for high expression of PRPP synthetase.

Another object of the present invention is to provide a novel microorganism transformed by the novel recombinant DNA.

Still another object of the present invention is to provide a process for producing nucleic acid related substances by using the novel microorganism.

These objects as well as other objects and advantages of the present invention will be apparent to those skilled in the art from the following description with reference to accompanying drawings.

## BRIEF EXPLANATION OF DRAWINGS

Fig. 1 shows a restriction map of the recombinant plasmid pPRS1 containing the gene (prs) coding for PRPP synthetase obtained in Example 1 (ii) hereinafter. In Fig. 1, the solid line represents the DNA fragment containing prs and the blank part represents pUC19.

Fig. 2 shows a restriction map of the deletion plasmid pPRS1505 obtained in Example 1 (iii) hereinafter. In Fig. 2, the solid line represents the DNA fragment containing a prs gene and the blank part represents the vector pHSG398. Further, the nucleotide sequence of the junctions are also shown.

Fig. 3 is a restriction map of pPSC32 obtained in Example 1 (iv) hereinafter. In Fig. 3, the solid line part represents the DNA fragment containing a prs gene, the black-colored part represents a SP promoter, the shaded part represents a chloramphenicol acetyltransferase gene (cat) and the blank part represents pBR322.

## SUMMARY OF THE INVENTION

According to the present invention, there is provided a recombinant DNA comprising DNA containing a phosphoribosyl pyrophosphate synthetase structural gene of a microorganism of the genus Bacillus, DNA containing a ribosome binding site and DNA containing a promoter sequence being capable of expression in a microorganism of the genus Bacillus, these DNA sequences being modified so as to enhance the expression of the phosphoribosyl pyrophosphate synthetase gene.

The DNA containing the promoter sequence is inserted into the PRPP synthetase gene so as to be located closely to and upstream from the DNA containing the ribosome binding site, which is followed by the open-reading frame of the PRPP synthetase gene.

Further, the present invention provides a vector containing the recombinant DNA, a novel microorganism of the genus Bacillus capable of producing nucleic acid related substances which are transformed with the recombinant DNA or vector, and a process for producing nucleic acid related substances using the microorganism of the genus Bacillus.

## DETAILED DESCRIPTION OF THE INVENTION

DNA containing a PRPP synthetase gene used in the present invention is preferably that comprising all of the DNA coding for the enzyme and some 5'-flanking and/or 3'-flanking regions of the DNA part. The DNA may also be that comprising a part of the enzyme gene, or that comprising a part of the enzyme gene and its 5'- or 3'- flanking region. Such a DNA is conveniently isolated from a chromosal DNA of a micoorganism by using recombinant DNA techniques, and examples of a host-vector system used for this purpose including the EK system can be suitably used.

Namely, as a host, there can be suitably used strains derived from Escherichia coli K-12, for example, Escherichia coli C600 (T. Maniatis et al., "Molecular Cloning, A. Laboratory Manual", Cold Spring Harbor Laboratory, published by Cold Spring Harbor Laboratory Press, 1982, page 504), Escherichia coli JA221 (IFO 14359, ATCC 33875), Escherichia coli MM294 (ATCC 33625) [Proc. Natl. Acad. Sci. USA, 73, 4174 (1976)] or derivatives thereof. As a vector, pBR322, pACYC184, pACYC177, pUC18, pUC19, pHSG396, pHSG298 and the like are suitably used.

Further, other host-vector systems such as of phage vectors, for example, Charon 4A (Molecular Cloning, page 31), EMBL3 and EMBL4 (J. Mol. Biol., 170, 827) and the like as a vector, and Escherichia coli DP50 supF (Molecular Cloning, page 504), Escherichia coli NM538 (J. Mol. Biol., 170, 827), Escherichia coli LE392 (J. Biol. Chem., 218, 97) and the like as a host can be used. As a matter of course, a host-vector system wherein a microbe of the genus Bacillus, for example, bacillus subtilis MI114 (Gene, 24, 255) or its mutant is used as the host and a plasmid having capability of autonomous replication in a microorganism of the genus Bacillus such as pUB110, pC194, pE194, pS194, pSA2100, pHY300PLK or the like is used as the vector can also be employed.

In principle, the origin of a donor of the DNA containing a PRPP synthetase structural gene is not specifically limited in so far as the nucleotide sequence of the DNA of the donor has high homology to that of a PRPP synthetase structural gene on the chromosome of a microorganism of the genus Bacillus to be used as a recipient as described hereinafter, and any microorganism can be used as the donor. Among them, when a microorganism of the genus Bacillus is used, so-called self-cloning is realized and it is preferred because of easy handling. Further, the resulting transformant is expected to be stable. Furthermore, the donor is not necessarily capable of producing the nucleic acid related substances.

Examples of such a DNA donor include microorganisms of the genus Bacillus such as Bacillus subtilis Bacillus pumulus and Bacillus licheniformis. For example, the following microbial strains can be used:

Bacillus subtilis No. 168 (BGSC 1A1)

Bacillus subtilis No. 115 (IFO 14187, FERM BP-1327)

Bacillus subtilis MI114 (Gene, 24, 255)

Bacillus pumilus ATCC 7061

Bacillus licheniformis ATCC 14580

wherein BGSC is The Bacillus Genetic Stock Center, IFO is Institute for Fermentation, Osaka, Japan and ATCC is The American Type Culture Collection, U.S.A.

The chromosome DNA can be isolated from the donor, according to conventional methods, for example, extraction of chromosome DNA with phenol (H. Saito and K. Miura, Biochim. Biophy. Acta., 72, 619). The chromosome DNA thus obtained is cleaved with a suitably selected restriction enzyme and ligated to a vector with a DNA ligase. The ligation mixture is used for transformation of a host having a deficiency in PRPP synthetase to select a transformant wherein the deficiency has been complemented. Thereby, the DNA containing a PRPP synthetase structure gene can be obtained. Alternatively, the DNA containing a PRPP synthetase structural gene can be cloned by using a part of the known DNA sequence of the PRPP synthetase structural gene of Bacillus subtilis [D. Nilsson et al., Mol. Gen. Genet., 218, 565 (1989)] as a probe and conducting colony hybridization or plaque hybridization methods. The transformation of the host can be conducted according to conventional methods. For example, in the case that the host strain is Escherichia coli, the transformation can be conducted according to the method described by S.N. Cohen et al. (Pro. Natl. Sci. USA, 69, 2110). In the case that the host is a microbe of the genus Bacillus, the transformation can be conducted according to a known method, for example, the method described by S. Chang and S.N. Cohen (Mol. Gen. Genet., 168, 115).

The large scale isolation of the DNA containing the structural gene from the transformant thus obtained can be conducted according to the method described in the above "Molecular Cloning", pages 86 to 93.

For construction and isolation of the novel recombinant DNA of the present invention from the DNA containing a PRPP synthetase gene thus obtained, conventional recombinant DNA techniques are conveniently employed and, for example, a host-vector system of Escherichia coli can be suitably used and, in general, the construction and isolation can be conducted according to the following steps.

In the following construction steps of the recombinant DNA of the present invention in Escherichia coli, in many cases, it is convenient to subclone a DNA fragment into a new vector or to ligate it by using a polylinker. For this purpose, it is preferable to conduct subcloning by utilizing, for example, a polylinker portion of pUC118 or pUC119 which is a vector of Escherichia coli because both the construction of the novel recombinant DNA and the determination of nucleotide sequence can be conveniently conducted.

The novel recombinant DNA of the present invention can be obtained from a plasmid containing a PRPP synthetase gene by using a suitable restriction enzyme and, if necessary, an enzyme having exonuclease activity which digests double stranded DNA from its terminal end, for example, BAL31 to cleave and delete the 5′-upstream region in the initiation codon of the open reading flame of the gene. To this is ligated a ribosome binding site, which is a sequence concerned in binding to the 16S ribosome RNA of the recipient, in the same direction at the 5′-upstream from the initiation codon of the open reading flame. Further, to this is ligated a promoter sequence having promoter activity such that it can express highly in a microbe of the genus Bacillus at the 5′-upstream from the ribosome binding site.

As for the ribosome binding site of the present invention, any DNA having a sequence complementary to $3'^{OH}UCUUUCCUCC5'$ can be used, when they are used as the recipient described hereinafter. The sequence of $3'^{OH}UCUUUCCUCC5'$ is the messenger RNA binding site of the 16S ribosome of microbes belonging to the genus Bacillus.

The sequence can be obtained either by cloning from chromosome DNA of microbes belonging to the genus Bacillus or by synthesizing it. The sequence present in the region upstream from the open-reading flame of the cloned PRPP synthetase gene described above may also be utilized without any modification.

For obtaining the DNA wherein only the open reading flame of the PPRP synthetase gene remains, or for obtaining the DNA wherein the open reading flame as well as the ribosome binding site located at 5′-upstream from the open reading flame remains, the kind of the rstriction enzyme, the amount of enzyme having exonuclease activity and the reaction time are suitably changed to obtain DNA fragments having different kinds of cleaved parts and size, and then the desired DNA fragment can be selected by suitable means as described hereinafter, for example, the determined nucleotide sequence of the fragment. Determination of the nucleotide sequence can be conducted by a known method, for example, subcloning the DNA fragment in a vector to be used for the determination of nucleotide sequence, for example, M13, pUC118 or pUC119 and then determining according to a known method such as the method of F. Sangar et al. (Proc. Natl. Acad. Sci. USA, 74, 5463).

The promoter sequence used in the present invention is not limited to a specific one in so far as it has a DNA sequence which can expressed in a recipient to be used in the later step, regardless of its origin, i.e., prokaryote, eukaryote or synthetic DNA.

Examples thereof include promoters derived from chromosomes of microorganisms of the genus Bacillus, phage of microorganisms of the genus Bacillus, or the plasmids having capability of autonomous replication in cells of microorganisms of the genus Bacillus.

Specific examples of such promoters are as follows:

```
AAAAGGTATTGACTTTCCCTACAGGGTGTGTAATAATTTATATACA

          SPO1-15 [Lee, G and Pero, J, J. Mol. Biol., 152

                  247 (1981)]

AAAAGTTGTTGACTTTATCTACAAGGTGTGGCATAATAATCTTAAC

          SPO1-26 [Lee et al., Mol. Gen. Genet., 180, 57

                  (1980)]

GAAAAGTGTTGAAAATTGT CGAACAGGGTGATATAATAAAAGAGTA

          φ29G3b [Marray, C.L. and Robinowtiz, J.C.,

                  J. biol. Chem., 257, 1053 (1982)]

GAAAAGGGTAGACAAACTATCGTTTAACATGTTATACTATAATAGAA

          φ29G2 [Yoshikawa, H. et al., Proc. Natl. Acad.

                  Sci. USA, 78, 1336 (1981)]

ATTAATGTTTGACAACTAT TACAGAGTATGCTATAATGGTAGTATC

          φ29A1 [Yoshikawa, H. et al., Proc. Natl. Acad.

                  Sci. USA, 78, 1336 (1981)]

TAATATCGTTGACATTATCCATGTCCGTTGTTAAGATAAACATGAA

          pur operon
```

Such a promoter can be cut out from DNA (plasmid) containing it with a suitable restriction enzyme, fractionated by, for example, agarose gel electrophoresis or polyacrylamide gel electrophoresis and then recovered. Alternatively, DNA having the nucleotide sequence of the above promoter can be synthesized according to the phosphoamidate method by using a commercially available DNA synthesizer (e.g., apparatus manufactured by Applied Biosystems Inc., U.S.A.) and following its protocol.

Thus, the DNA of the present invention has been constructed and it is subjected to transformation of the recipient. In this case, when a marker gene, e.g., a drug resistance gene, having capability of expressing in a microorganism of the genus Bacillus is further ligated to the DNA of the present invention in advance, in some cases, selection of a transformant of the recipient as described hereinafter can be conveniently conducted.

Further, in this case, when a part of the 5'-flanking region of the PPRP synthetase gene is ligated to the 5'-upstream from the ligated promoter in advance, it is advantageous because double cross over type recom-

EP 0 465 132 A2

bination is caused upon recombination on the chromosomes of the recipient as described hereinafter.

In order to obtain a large amount of the novel recombinant DNA of the present invention, a host microorganism is transformed with the above ligation mixture, followed by collecting a transformant grown on a selective medium, for example, in the case of utilizing drug resistance as a selection marker, a medium containing the corresponding drug. A large amount of the recombinant DNA can be prepared from the transformant according to a method which itself has been known, such as the method described in pages 86 to 93 of the above "Molecular Cloning".

Hereinafter, a method for the derivation of the novel microorganism by transformation of a recipient with the novel recombinant DNA thus obtained is illustrated.

As the recipient for this transformation, any microorganism having capability of transformation, i.e., a property such as DNA provided outside of the cells can be incorporated therein and recombination is caused at a part on its chromosome having high homology to the nucleotide sequence of the DNA to change its inherent character, can be used. The recipient is not necessarily identical with the donor in so far as the nucleotide sequence of the PRPP synthetase gene on its chromosome DNA has high homology to that of the PRPP synthetase gene contained in the donor DNA. For the derivation of a transformant having high capability of producing nucleic acid related substances, microorganisms of the genus Bacillus, particularly, Bacillus subtilis, Bacillus pumilus, Bacillus licheniformis and the like are suitable from the viewpoint that they are known to accumulate purine nucleotide related substances (e.g, inosine, guanosine, etc.) and pyrimidine nucleotide related substances (e.g., uridine, cytidine, etc.) and the like, they have capability of transformation, they have no pathogenicity, and they have been used safely in fermentation industries.

Further, in many cases, a much improved result in the production of nucleic acid related substances can be obtained, when a microorganism wherein one or more known characters advantageous for accumulation of the nucleic acid related substances, for example, nucleic acid analogue-resistance such as 8-azaguanine resistance, nucleotide phosphorylase deficiency, 5′-guanylic acid reductase deficiency, folic acid antagonist resistance are provided in the above microorganism which is used as the recipient.

Examples of the recipient of the genus Bacillus include:

Bacillus subtilis BM1032 (IFO 14559, FERM BP-1242)

Bacillus subtilis IA3 (BGSC No. 1A3)

Bacillus subtilis NA-6011 (IFO 14189, FERM BP-291)

Bacillus subtilis NA-6012 (IFO 14190, FERM BP-292)

Bacillus subtilis NA-6128 (IFO 14373, FERM BP-617)

Bacillus subtilis NA-7821 (IFO 14368, FERM BP-618)

Bacillus subtilis ATCC 19221

Bacillus subtilis ATCC 14662

Bacillus pumilus (FERM P-2116)

Bacillus pumilus (FERM P-4832)

Bacillus pumilus (FERM P-4829)

Bacillus licheniformis IFO 12485

The novel recombinant DNA of the present invention is used for transformation of a microorganism of the genus Bacillus. In this case, it is possible to use it in an integrated form in a plasmid, or in the form of a linear DNA obtained by cleavage of a plasmid or cutting out of the DNA of the present invention. The transformation of a microorganism of the genus Bacillus with the DNA fragment can be conducted according to a known method [C. Anagnostopoulos and J. Spizizen, J. Bacterial., 81, 741 (1961); etc.].

The selection of the transformant can be readily conducted by, for example, in the case of using a drug resistant gene as a selection marker, cultivation on an agar plate containing the corresponding drug.

Whether the transformant thus obtained is the novel microorganism having the desired characteristics or not can be readily identified by measuring the PRPP synthetase activity. Namely, the novel microorganism transformed with the recombinant DNA of the present invention of which PPRP synthetase gene expression is enhanced has high PRPP synthetase activity in comparison with its parent strain.

A typical example of the transformant is Bacillus subtilis BM3032 (IFO 15058, FERM BP-2987) obtained in the Example as described hereinafter.

As a matter of course, by selecting the promoter and the recipient, various transformants can be readily derived according to the method described herein.

For producing nucleic acid related substances by using the transformant obtained in the present invention, a conventional fermentation process for producing the nucleic acid related substances can be employed.

Examples of nucleic acid related substances include purine nucleotide related substances (e.g., inosine, guanosine, etc.), pyrimidine nucleotide related substances (e.g., uridine, cytidine, etc.) and the like. Particularly, purine nucleotide related substances such as inosine, guanosine and the like are preferred.

6

For fermentation, culture media containing carbon sources, nitrogen sources, metal ions and, if necessary, other nutrients such as amino acids, nucleic acids, vitamines and the like can be used. Examples of carbon sources include glucose, sucrose, maltose, starch, saccharified starch, molasses and the like. Examples of nitrogen sources include organic nitrogenous substances such as peptone, corn steep liquor, soybean meal, dried yeast, urea and the like; and further inorganic nitrogenous substances such as ammonium salts of sulfuric acid, nitric acid, hydrochloric acid, carbonic acid and the like, gaseous ammonia and aqueous ammonia and the like. They can be used alone or in combination thereof.

As other nutrients, various inorganic salts, amino acids, vitamins and the like which are essential to the growth of the microorganism are appropriately selected and they are used alone or in combination thereof.

Furthermore, if necessary, an antifoaming agent, a surfactant and the like such as silicone oil, polyalkylene glycol ether and the like can be added to the culture medium.

The cultivation is normally conducted under aerobic conditions such as shaking culture or aeration submerged culture and the like. Preferably, the pH of the culture medium is within the range of between 4 and 9. If the pH varies during cultivation, sulfuric acid, calcium carbonate, sodium hydroxide, gaseous ammonia, aqueous ammonia and the like can be added to adjust the pH to within the above preferred range. The cultivation temperature is normally selected from the range of 20 to 45°C so that the temperature is suitable for the growth of the microorganism used and the accumulation of the desired nucleic acid related substances. The cultivation is continued until the amount of the nucleic acid related substances accumulated substantially reaches the maximum. Normally, the desired result can be obtained by cultivation for 24 to 144 hours.

For separating and isolation of the nucleic acid related substances from the culture, conventional isolation and purification methods, for example, precipitation, chromatography using an ion exchange resin or activated charcoal and the like can be employed.

As described hereinabove, according to the present invention, the industrially advantageous production of inosine and/or guanosine which are the raw materials for the production of important flavor substances, 5'-inosinic acid and/or 5'-guanylic acid can be realized with high yield.

Namely, a novel microorganism having an improved activity of PRPP synthetase which is an enzyme concerned in the synthesis of an important intermediate for the production of nucleic acid related substances, PRPP, can be obtained by using a microorganisms of the genus Bacillus capable of producing nucleic acid related substances as a recipient and transforming it with the DNA of the present invention.

The novel microorganism can constantly produce a large amount of the nucleic acid substances.

The following Examples further illustrate the present invention in detail but are not to be construed to limit the scope thereof. The operations in the Examples were conducted according to the following methods (1) to (8) unless otherwise stated.

(1) Digestion of DNA with restriction enzyme

Buffer solutions for restriction enzymes

(manufactured by Takara Shuzo Co., Ltd., Japan) used were those specified by the manufacturer. The digestion was conducted by using the enzyme of 10 units/1 μg DNA at 37°C. Then, the reaction mixture was extracted with phenol saturated with a TE buffer solution [1O mM Tris-HCl (pH 7.5) and 1 mM EDTA). To the extract were added 1/10 volumes of 3 M sodium acetate (pH 6) and then 2.5 volumes of ethanol and the mixture was centrifuged to precipitate DNA.

(2) Extraction of a large amount of plasmid

The extraction was conducted according to the method described in pages 86 to 93 of "Molecular Cloning". Namely, Escherichia coli harboring plasmid DNA was inoculated into LB medium (250 ml; 10 g/liter of bactotryptone, 5 g/liter of yeast extract and 5 g/liter of sodium chloride) and cultivated overnight. Then, the microbial cells were collected and washed with TE buffer solution. To the washed cells was added lysozyme, followed by the addition of 0.2 N sodium hydroxide solution containing 1% sodium lauryl sulfate to cause lysis of the cells. After addition of 5 M potassium acetate, a supernatant containing the plasmid was obtained by centrifugation. To the supernatant was added 0.6 parts by volume of isopropanol to precipitate the plasmid DNA and, after washing with ethanol, it was dissolved in TE buffer solution. To this was added cesium chloride so that the specific gravity became 1.60, followed by the addition of ethidium bromide so that the final concentration became 600 μg/ml. The mixture was centrifuged at 50,000 rpm at 20°C for 12 hours by an ultracentrifuge (Rotor $V_{65}$Ti) and a band of plasmid detected by ultraviolet rays was taken out. After removing ethidium bromide by extraction with n-butanol, ethanol precipitation was conducted.

7

(3) Transformation of Escherichia coli

The transformation was conducted according to the method described in page 250 of "Molecular Cloning". Escherichia coli was inoculated into 3 ml of LB medium and cultivated overnight. A part of the culture broth (1 ml) was inoculated into 100 ml of LB medium and subjected to shaking culture at 37°C until the number of the microbial cells became about $5 \times 10^7$ cells/ml. Then, the cells were collected and they were suspended and ice-cooled for 15 minutes by addition of ice-cooled sterilized water (50 ml) containing 50 mM calcium chloride and 10 mM Tris-HCl buffer (pH 8). The cells were collected by centrifugation and they were again suspended in the above aqueous solution (6.7 ml) and DNA was added to 0.2 ml portion of this solution. The mixture was ice-cooled for 30 minutes. To this was added 0.8 ml of LB medium and the mixture was incubated at 37°C for 1 hour. Then, the culture was spread over a medium of LB agar plate containing a drug and incubated at 37°C overnight.

(4) BAL31 exonuclease digestion treatment

BAL31 nuclease digestion treatment was conducted to digest both double strands of DNA from both terminal ends. Namely, DNA (10 µg) treated with a restriction enzyme was dissolved in BAL31 buffer solution [100 µl; 12 mM calcium chloride, 12 mM magnesium chloride, 200 mM sodium chloride, 20 mM Tris-HCl (pH 8) and 2 mM EDTA] and BAL31 exonuclease (1 unit, manufactured by Takara Shuzo Co., Ltd., Japan) was added to the mixture. The mixture was incubated at 30°C. After digestion, phenol extraction and ethanol precipitation were conducted. Then, in order to convert the digested cohesive ends of DNA into blunt ends, the above DNA was suspended in a buffer solution for T4 DNA polymerase [20 µl; containing 33 mM Tris-Acetic acid (pH 7.9), 10 mM magnesium acetate, 0.5 mM dithiothreitol, 66 mM potassium acetate, 0.01 % BSA, and 0.1 mM dATP, dGTP, dCTP and dTTP] and T4 DNA polymerase (5 units, manufactured by Takara Shuzo Co., Ltd., Japan) was added to the mixture. The mixture was incubated at 37°C for 30 minutes. After incubation, phenol extraction and ethanol precipitation were conducted.

(5) Ligation of DNA

A DNA ligation kit (manufactured by Takara Shuzo Co., Ltd., Japan) was used for ligation of 2 to 3 DNA fragments. The ligation conditions followed were those specified by the manufacturer.

(6) Transformation of Bacillus subtilis

The transformation was conducted according to the method described by Dubnou et al. Namely, a microorganism was inoculated into LB medium (5 ml) and incubated at 37°C overnight. A 0.5 ml portion thereof was transferred to SPI medium (20 ml; containing 1.4% dipotassium phosphate, 0.6% monopotassium phosphate, 0.2% ammonium sulfate, 0.1% sodium citrate, 0.02% magnesium sulfate, 0.5% glucose, 0.02% Casamino acid, 0.1% yeast extract, 50 µg/ml of tryptophan and 50 µg/ml of leucine) and incubated at 37°C for 4 hours. Then, a 10 ml portion of the latter culture broth was transferred to SPII medium (100 ml; containing 1.4% dipotassium phosphate, 0.2% ammonium sulfate, 0.1% sodium citrate, 0.02% magnesium sulfate, 0.5% glucose, 75 µg/ml of calcium and 508 µg/ml of magnesium chloride) and incubated at 37°C for 90 minutes. To a 1 ml portion thereof was added 1 µg of a DNA and the mixture was shaken at 37°C for 30 minutes. Then, it was spread on LB agar plate containing a drug and incubated at 37°C overnight.

(7) Colony hybridization

The transformed colonies were inoculated on a nylon filter (colony/plaque screen NFF-978X, manufactured by E.I. du Pont de Nemours & Co., Inc., U.S.A.) placed on LB agar plate containing 50 µg/ml of ampicillin, and on a master plate of LB agar containing ampicillin, respectively and they were incubated at 37°C overnight.

Then, this nylon filter was removed from the plate and dipped in 0.5 N sodium hydroxide solution for 10 minutes and further dipped in 1M Tris-HCl (pH 7.5) for 10 minutes. Then the filter was dried at room temperature.

On the other hand, a probe was prepared. The preparation was conducted by using MEGALABEL (manufactured by Takara Shuzo Co., Ltd., Japan) according to the protocol specified by the manufacturer.

The method for hybridization followed was according to the protocol specified by the manufacturer.

(8) Determination of nucleotide sequence

The determination of the nucleotide sequence was conducted by using SEQUENASE (manufactured by Toyo Boseki Kabushiki Kaisha, Japan) according to the method specified by the manufacturer.

The IFO numbers used herein is the accession number in the Institute for Fermentation, Osaka and the FERM BP number is the accession number at the Fermentation Research Institute, Agency of Industrial Science and Technology (FRI) under Budapest treaty.

Example 1

(i) Preparation of chromosome DNA

Bacillus subtilis No. 115 [IFO 14187, BP-1327 (deposited March 28, 1987)] was inoculated into LB medium (40 ml) and incubated at 37°C overnight. Chromosomal DNA (5 mg) was obtained from the microbial cells by chromosomal DNA extraction method with phenol.

(ii) Cloning of PPRP synthetase gene (prs)

The chromosomal DNA (10 μg) prepared in the above (i) was digested with BclI, fractionated by agarose gel electrophoresis, and then a DNA of about 3.6 to 4.5 kb was recovered by using a unidirectional electroeluter (manufactured by International Biotechnologies Inc.).

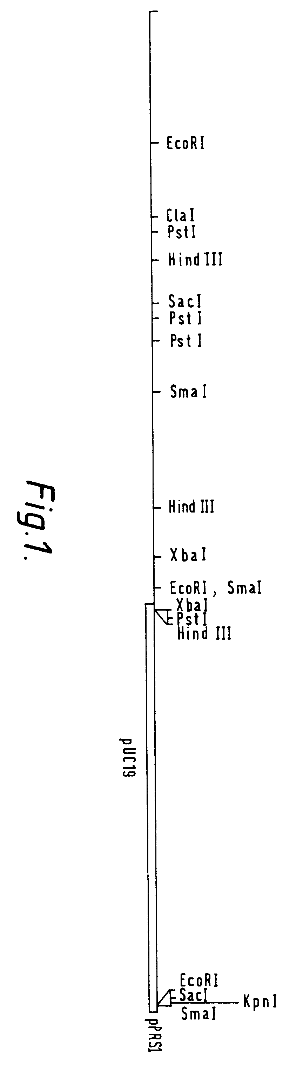

On the other hand, 0.5 μg of pUC19 was digested with BamHI, mixed with the above DNA fragment and ligated. Then, Escherichia coli JM109 (manufactured by Takara Shuzo Co., Ltd., Japan) was transformed to select an ampicillin resistant strain. Colony hybridization was conducted by selecting colonies of about 200 strains and using a synthetic oligonucleotide (5′-CCAGACCATGGCGGTGTGACACGTGCCCGC-3′) as a probe. Plasmid pPRS1 was obtained from, among the strains which hybridized with the probe, one strain [Escherichia coli JM109 (pPRS1)]. The restriction cleavage map of this pPRS1 is shown in Fig. 1.

(iii) Preparation of deletion plasmid pPRS1505

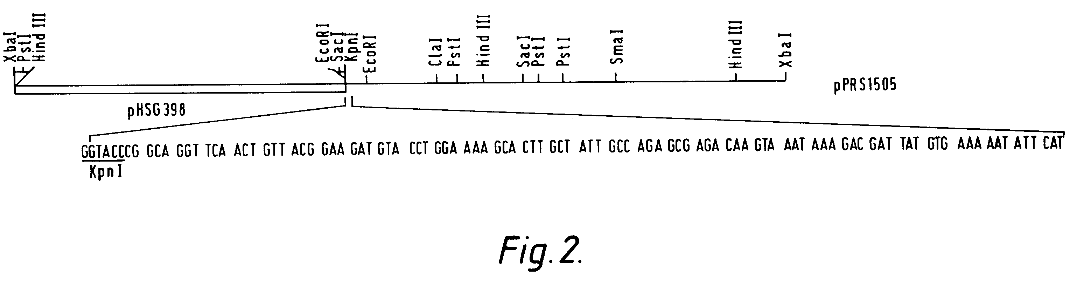

After 10 μg of pPRS1 obtained in the above (ii) was digested with KpnI, BAL31 digestion treatment for 10 minutes was conducted. Then, it was completely digested with XbaI and fractionated by agarose gel electrophoresis to recover DNA of about 3.0 kb. This DNA was ligated to 0.5 μg of pHSG398 (manufactured by Takara Shuzo Co., Ltd., Japan) which had been subjected to double digestion with SmaI and XbaI. Then, Escherichia coli JM109 was transformed to select a chloramphenicol (30 μg/ml) resistant strain. As a result, plasmid pPRS1505 as shown in Fig. 2 was obtained.

(iv) Preparation of pPSC32

pPRS1505 (1 μg) prepared in the above (iii) was digested with KpnI and then partially digested with HindIII, and further subjected to agarose gel electrophoresis to recover a DNA fraction of about 3.0 kb. Likewise, pSP19 (0.5 μg, EP-A 412688) was subjected to double digestion with EcoRI and KpnI and fractionated by agarose gel electrophoresis to recover a DNA fraction of about 0.1 kb. pCAT 15 (0.5 μg, Japanese Patent Laid Open Publication No. 82096/1987) was subjected to double digestion with HindIII and BamHI and fractionated by agarose gel electrophoresis to recover a DNA fraction of about 1.0 kb. pBR322 (0.5 μg) was subjected to double digestion with EcoRI and BamHI and fractionated by agarose gel electrophoresis to recover a DNA fraction of about 3.9 kb.

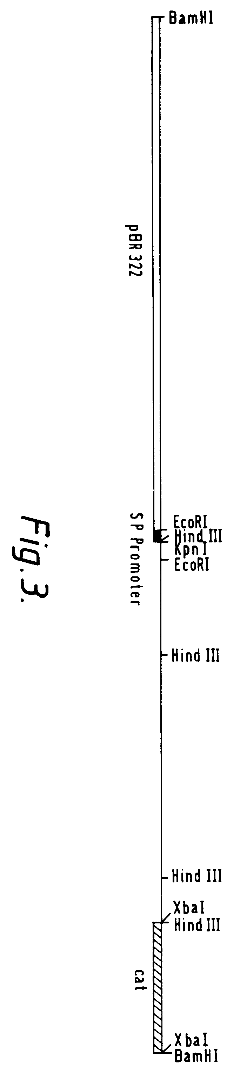

Then, these four DNA fragments were mixed and ligated, and then Escherichia coli JM109 was transformed by them. An ampicillin resistant was selected to obtain Escherichia coli JM109 (pPSC32). The restriction cleavage map of its plasmid, pPSC32. is shown in Fig. 3.

Example 2

(i) Transformation of inosine and/or guanosine producer, Bacillus subtilis BM1032, with pPSC32

Bacillus subtilis BM1032 (IFO 14459, FERM BP-1242) was transformed with 1 μg of pPSC32 prepared as In Example 1 (iv) to obtain chloramphenicol (10 μg/ml) resistant strains. Among them, one strain was named as Bacillus subtilis BM3032. This strain has been deposited at IFO since June 20, 1990 under the accession number of IFO 15058, and deposited at FRI under Budapest Treaty since June 25, 1990 under the the accession number FERM BP-2987.

(ii) Productivity of inosine and guanosine with Bacillus subtilis BM3032

A loopful of Bacillus subtilis BM3032 grown on 5 ml of LB medium was inoculated into 20 ml of the medium as shown in Table 1 and incubated at 37°C for 53 hours. When the amount of inosine and guanosine accumulated in the culture broth at the end of the fermentation was determined by high performance liquid chromatography, 14 mg/ml of inosine and 2.5 mg/ml of guanosine were accumulated. On the other hand, when Bacillus subtilis BM1032 was cultivated under the same conditions as described above, only 8.0 mg/ml of inosine and 1.1 mg/ml of guanosine were accumulated in the culture broth.

Table 1

| Component of medium | Concentration (g/liter) |
| --- | --- |
| Glufinal | 120 |
| Monosodium glutamate | 12 |
| Ammonium nitrate | 20 |
| DL-α-alanine | 0.3 |
| Casamino acid | 10 |
| Tryptophan | 0.2 |
| Leucine | 0.2 |
| Ribonucleic acid (RNA) | 2 |
| Biotin | 0.0002 |
| Monopotassium phosphate | 2 |
| Potassium chloride | 1 |
| Magnesium sulfate | 1 |
| Ferrous sulfate | 0.005 |
| Manganese sulfate | 0.01 |
| Calcium carbonate | 15 |
| pH | 7.5 |

(iii) Measurement of PRPP synthetase activity

Bacillus subtilis BM3032 was cultivated in SPI medium containing adenine overnight and the cells were collected and washed. The cells were broken and, by using its supernatant, PRPP synthetase activity was measured according to a method described by K. F. Jensen et al. [K. F. Jensen et al., Analytical Biochemistry, 98, 254-263 (1979)].

As a result, the transformant of the present invention, <u>Bacillus</u> <u>subtilis</u> BM3032, showed 2.8 times higher PRPP synthetase activity than that of <u>Bacillus</u> <u>subtilis</u> BM 1032 which was used as a control strain.

Sequence Listing

SEQ ID NO: 1

SEQUENCE TYPE: Nucleotide

SEQUENCE LENGTH: 104 base pairs

STRANDEDNESS: single

TOPOLOGY: linear

MOLECULE TYPE: chromosomal DNA

ORIGINAL SOURCE

ORGANISM:  bacillus

PROPERTIES: DNA containing a PRPP synthetase structural gene

GGTACCCGGC AGGTTCAACT GTTACGGAAG ATGTACCTGG AAAAGCACTT GCTATTGCCA      60

GAGCGAGACA AGTAAATAAA GACGATTATG TGAAAAATAT TCAT                      104

**Claims**

1. A recombinant DNA comprising DNA containing a phosphoribosyl pyrophosphate synthetase structural gene of a microorganism of the genus Bacillus, DNA containing a ribosome binding site and DNA containing a promoter sequence being capable of expression in a microorganism of the genus Bacillus, these DNA sequences being modified so as to enhance the expression of the phosphoribosyl pyrophosphate synthetase gene.

2. A recombinant DNA according to claim 1, wherein the DNA containing the promoter sequence is located closely to and upstream from the DNA containing the ribosome binding site.

3. A recombinant DNA according to claim 1, wherein the DNA sequence is modified at the ribosome binding site.

4. A recombinant DNA according to claim 1, wherein the DNA sequence is modified at the promoter sequence.

5. A recombinant DNA according to claim 4, wherein the promoter sequence is exchanged with another promoter sequence being capable of expression.

6. A recombinant DNA according to claim 5, wherein the other promoter sequence is a promoter sequence derived from a chromosomal DNA of a microorganism of the genus Bacillus or a phage of a microorganism of the genus Bacillus.

7. A recombinant DNA according to claim 1, wherein a donor of the structural gene is a microorganism of Bacillus subtilis.

8. A recombinant DNA according to claim 7, wherein the donor is Bacillus subtilis No. 115 (FERM BP-1327).

9. A recombinant DNA according to claim 1, wherein a donor of the structural gene is a microorganism of Bacillus pumilus.

10. A recombinant DNA according to claim 1, wherein a donor of the structural gene is a microorganism of Bacillus licheniformis.

11. A vector containing the recombinant DNA according to any of claims 1 to 6.

12. A microorganism of the genus Bacillus being capable of producing nucleic acid related substances which is-transformed with the recombinant DNA according to any of claims 1 to 6.

13. A microorganism of the genus Bacillus being capable of producing nucleic acid related substances which is transformed with the vector according to claim 11.

14. A microorganism according to claim 12 being Bacillus subtilis.

15. A microorganism according to claim 14 being Bacillus subtilis BM3032 (FERM BP-2987).

16. A method for producing nucleic acid related substances which comprises cultivating the microorganism of the genus Bacillus according to either of claims 12 or 13 to form and accumulate, the nucleic acid related substances, and collecting the nucleic acid related substances.

17. A method according to claim 16, wherein the microorganism is Bacillus subtilis.

18. A method according to claim 17, wherein the microorganism is Bacillus subtilis Bm3032 (FERm BP-2987).

Fig.1.

EcoRI

ClaI
PstI

HindIII

SacI
PstI

PstI

SmaI

HindIII

XbaI

EcoRI, SmaI
XbaI
PstI
HindIII

pUC19

EcoRI
SacI
SmaI
KpnI

pPRS1

XbaI
PstI
Hind III

EcoRI
SacI
KpnI
EcoRI

ClaI
PstI

Hind III

SacI
PstI

PstI

SmaI

Hind III

XbaI

pPRS1505

pHSG398

GGTACCCG GCA GGT TCA ACT GTT ACG GAA GAT GTA CCT GGA AAA GCA CTT GCT ATT GCC AGA GCG AGA CAA GTA AAT AAA GAC GAT TAT GTG AAA AAT ATT CAT

KpnI

*Fig.2.*

Fig.3.